# EUROPEAN PATENT APPLICATION

(11) **EP 4 273 874 A1**
(43) Date of publication of application: **08.11.2023**
(21) Application number: 21915835.9
(22) Date of filing: 29.12.2021
(51) Int. Cl.: G16H 40/20, G16H 10/20, G06Q 10/08

(54) **CLINICAL SUPPLIES MANAGEMENT METHOD AND DEVICE USING SAME**

(30) Priority: 30.12.2020 KR 20200188193
(71) Applicant: Celltrion Inc., Incheon 22014 (KR)
(72) Inventor: PARK, Sung Ah, Incheon 22014 (KR); KOH, Keun Ho, Incheon 22014 (KR); ROH, Eun Sol, Incheon 22014 (KR); MOON, Dae Man, Incheon 22014 (KR); PARK, Young Shin, Incheon 22014 (KR); AHN, Sang Hoon, Incheon 22014 (KR); OH, Se Jin, Incheon 22014 (KR); WI, Sung Mok, Incheon 22014 (KR)
(74) Representative: EIP
(86) International application number: PCT/KR2021/020195
(87) International publication number: WO 2022/146038

(57) **Abstract**

Disclosed herein a method for clinical supplies management. The method includes registering a user associated with a clinical trial according to user types, receiving a request for providing clinical supplies related to the clinical trial, receiving providing completion information for the clinical supplies, and updating clinical supplies information corresponding to the providing completion information.

## Description

### Technical Field

The present disclosure relates to a clinical supplies management system, in particular to a method, apparatus and computer program for performing management for the conclusion of a contract, the execution of a contract and the termination of a contract for clinical supplies online.

This application claims the benefit of Korean Patent Application No. 10-2020-00188193, filed December 30, 2020, which is hereby incorporated by reference in its entirety into this application.

### Background Art

Clinical trial(Clinical Study) is test or study conducted on humans to prove the safety and effectiveness of medicines and medical devices. For example, in the case of pharmaceutical clinical trial, it is conducted to assess the pharmacokinetics, pharmacodynamics, pharmacology, and clinical efficacy of a drug and to investigate adverse events.

The phases of pharmaceutical clinical trials consist of four stages. In Phase I (Phase 1 clinical trial), primarily a small number of healthy adults are involved to collect data on drug absorption, distribution, metabolism, excretion, and evaluate safety. In Phase II (Phase 2 clinical trial), the focus is on evaluating the appropriate dosage range (optimal dose, etc.) and administration methods. Phase III (Phase 3 clinical trial) typically involves hundreds or more patients to ultimately validate the drug's efficacy and safety. In Phase IV (Phase 4 clinical trial), post-marketing surveillance is conducted to track adverse effects, reassess safety, and perform additional studies.

The key stakeholders involved in clinical trials can be broadly categorized as i) clinical trial personnel, ii) storage/transport personnel for clinical supplies, and iii) pharmaceutical company officials. Effective execution of clinical trials requires efficient communication and management of clinical supplies among these stakeholders.

In particular, Investigator Initiated Clinical Trials (IIT), which are driven by researchers, are not directly associated with revenue generation for pharmaceutical companies. Therefore, in the domestic context, IIT is not as active as Sponsor Initiated Clinical Trial (SIT) where the sponsor takes the lead. However, the importance of IIT is increasing day by day as they are deemed necessary for public welfare.

However, there is a lack of an efficient clinical supplies management system that enables the key stakeholders involved in clinical trials to communicate effectively and coordinate with each other for efficient management of clinical supplies. This situation leads to inconveniences for those conducting and supervising clinical trials or providing clinical supplies for such trials. In particular, it is challenging for professors or researchers at university hospitals, who are the primary drivers of Investigator Initiated Clinical Trials, to independently manage the supply of clinical materials. This poses a significant obstacle to the activation of investigator-initiated clinical trials.

### Disclosure

### Technical Problem

An object of the present disclosure is to effectively manage clinical supplies through an online platform so that the main entities involved in clinical trials can efficiently conduct clinical trials.

Another object of the present disclosure is to contribute to the activation of Investigator Initiated Clinical Trials by enabling clinical trial entities (researchers, subjects of clinical trial performance and supervision) such as professors at university hospitals to take the lead in managing the supply of clinical supplies.

Another object of the present disclosure is to share the cumulative consumption status of the total clinical quota with all entities participating in clinical trials related to the supplies, so that clinical supplies management optimized for Investigator Initiated Clinical Trials is possible.

Another object of the present disclosure is to increase the convenience of supplies management of Investigator Initiated Clinical Trials by making it easy for researchers to register new clinical participants.

Another object of the present disclosure is to facilitate the management of supplies for Investigator Initiated Clinical Trials, by enabling the contracting entities to easily verify the validity of the information printed on the packaging of clinical supplies.

### Technical Solution

In order to accomplish the above objects, a method for clinical supplies management according to an embodiment of the present disclosure includes registering a user associated with a clinical trial according to user types, receiving a request for providing clinical supplies related to the clinical trial, receiving providing completion information for the clinical supplies, and updating clinical supplies information corresponding to the providing completion information.

Here, the method for clinical supplies management may further include providing supply consumption status sharing interface for sharing the consumption status of the entire supplies allocated to the clinical trial to participants involved in the clinical trial.

Here, the supplies consumption status sharing interface may include graphs representing the total cumulative order quantities of the entire participants involved in the clinical trial, the total cumulative delivery completion quantities of the entire participants, and the total delivery quantities in progress of the entire participants.

Here, the user types may include study center type, study monitor type, depot type, pharmaceutical general type, and pharmaceutical administrator type.

Here, the supply consumption status sharing interface may be provided only for user types other than the depot type among the user types.

Here, the method for clinical supplies management may further include receiving a secondary user registration request for registering another user associated with the clinical trial by users corresponding to the study center type or the study monitor type.

Here, the providing completion information may include temperature excursion information and physical damage information corresponding to the clinical supplies.

Here, the method for clinical supplies management may further include receiving contract information corresponding to the contract related to the clinical trial.

Here, the method for clinical supplies management may further include requesting confirmation of variable text information to be displayed on the packaging of the clinical supplies.

Here, the confirmation of variable text information may be requested from the pharmaceutical administrator type user to the pharmaceutical general type user. Here, the variable text information may include information on individuals who have received approval for the clinical trial plan of the clinical trial.

Here, the method for clinical supplies management may further include receiving a return request corresponding to the clinical supplies. Here, the return request may include information on the reason for return, information on whether the transportation temperature is controlled during return, departure point information, and supplies information to be returned.

Here, the information on the reason for return may include any one or more of the clinical trial termination, expiration date expired, breakage/ temperature excursion, and recall due to drug abnormalities.

Also, a computer program or a recording medium storing a computer program according to an embodiment of the present disclosure may be for executing the clinical supplies management method described above.

Also, an apparatus for clinical supplies management of the present disclosure includes a processor that registers a user associated with a clinical trial according to user types, receives a request for providing clinical supplies related to the clinical trial, receives providing completion information for the clinical supplies, and updates clinical supplies information corresponding to the providing completion information and a memory that stores the clinical supplies information.

Here, the processor may provide supply consumption status sharing interface for sharing the consumption status of the entire supplies allocated to the clinical trial to participants involved in the clinical trial.

Here, the supply consumption status sharing interface may include graphs representing the total cumulative order quantities of the entire participants involved in the clinical trial, the total cumulative delivery completion quantities of the entire participants, and the total delivery quantities in progress of the entire participants.

Here, the user types may include study center type, study monitor type, depot type, pharmaceutical general type, and pharmaceutical administrator type.

Here, the supply consumption status sharing interface may be provided only for user types other than the depot type among the user types.

Here, the processor may receive a secondary user registration request for registering another user associated with the clinical trial by users corresponding to the study center type or the study monitor type.

Here, the processor may request confirmation of variable text information to be displayed on the packaging of the clinical supplies. Here, the confirmation of variable text information may be requested from pharmaceutical administrator type user to pharmaceutical general type user. Here, the variable text information may include the information on individuals who have received approval for the clinical trial plan of the clinical trial.

### Advantageous Effects

According to the present disclosure, clinical supplies can be effectively managed through an online platform so that the main entities involved in clinical trials can efficiently conduct clinical trials.

Also, the present disclosure can contribute the activation of Investigator Initiated Clinical Trials by enabling clinical trial entities (researchers, subjects of clinical trial performance and supervision) such as professors at university hospitals to take the lead in managing the supply of clinical supplies.

Also, the present disclosure allows the cumulative consumption status of the total clinical quota to be shared by all entities participating in clinical trials related to the supplies, so that clinical supplies management optimized for Investigator Initiated Clinical Trials is possible.

Also, the present disclosure increases the convenience of supplies management of Investigator Initiated Clinical Trials by making it easy for researchers to register new clinical participants.

Also, the present disclosure facilitates the management of supplies for Investigator Initiated Clinical Trials, by enabling the contracting entities to easily verify the validity of the information printed on the packaging of clinical supplies.

### Description of Drawings

FIG. 1 illustrates five types of users involved in clinical trials.
FIG. 2 illustrates an example of a user page provided to a user of a study center type or a study monitor type.
FIG. 3 illustrates an example of a user page provided to a user of the depot type.
FIG. 4 illustrates an example of a user page provided to a user of the pharmaceutical general type.
FIG. 5 illustrates an example of a user page provided to a user of the pharmaceutical administrator type.
FIGS. 6a to 6f illustrates other examples of a user page provided to a user of the pharmaceutical administrator type.
FIG. 7 illustrates an example of signing a contract related to a clinical trial.
FIG. 8 illustrates an example of an interface screen provided to the user of the pharmaceutical general type for applying a contract.
FIG. 9 illustrates an example of variable text.
FIG. 10 illustrates the situation that is different between the contracting entity and the practitioner of the contract related to the clinical trial.
FIG. 11 illustrates an example of user membership registration of a user corresponding to a practitioner.
FIG. 12 illustrates an example of the depot type user registration.
FIG. 13 illustrates an example in which a packaged drug is released.
FIG. 14 illustrates an example of supplying items using a clinical supplies management apparatus according to an embodiment of the present disclosure.
FIG. 15 is an example of an interface page provided to a user of a study center type for ordering items.
FIG. 16 illustrates an example of returning items using a clinical supplies management apparatus according to an embodiment of the present disclosure.
FIG. 17 illustrates an example of an overall process of managing supplies using a clinical supplies management apparatus according to an embodiment of the present disclosure.
FIG. 18 is an operation flow diagram illustrating an example of a clinical supplies management method according to an embodiment of the present disclosure.
FIG. 19 is a block diagram illustrating a clinical supplies management system according to an embodiment of the present disclosure.
FIG. 20 illustrates a computer system according to an embodiment of the present disclosure.

### Mode for Invention

Descriptions of the following exemplary embodiments refer to the attached drawings in which specific embodiments are illustrated by way of example. These embodiments are described in detail so that those having ordinary knowledge in the technical field to which the present disclosure pertains can easily practice the present disclosure. Accordingly, the shapes, sizes, etc. of components in the drawings may be exaggerated to make the description clearer.

Hereinafter, desirable embodiments according to the present disclosure will be described in detail with reference to the attached drawings.

Investigator initiated clinical trials (IIT) proceeds with the flow of signing a contract between an investigator and a pharmaceutical company for clinical trials, fulfilling the concluded contract (including drug supply), and terminating the contract. In order to enable effective management of supplies related to clinical trials through online platforms, it is necessary to classify users related to clinical trials into appropriate types and provide appropriate services according to the classified user types.

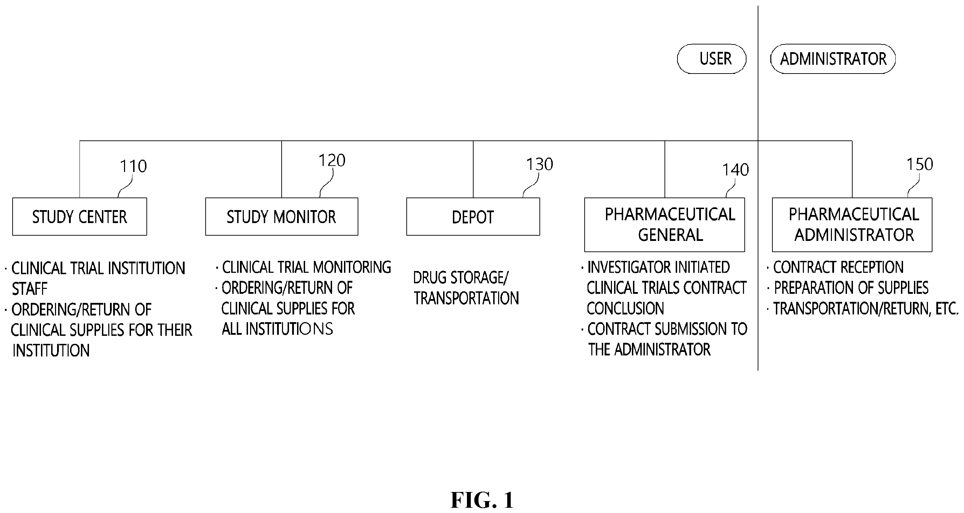

FIG. 1 illustrates five types of users involved in clinical trials.

Referring to FIG. 1, users related to clinical trials are classified into five types: study center type 110, study monitor type 120, depot type 130, pharmaceutical general type 140, and pharmaceutical administrator type 150.

The study center type 110 may be a staff member of a clinical trial institution such as an investigator or a professor, and they can request the ordering or return of clinical supplies for their own institution. That is, the user of the study center type 110 may be a user belonging to a clinical trial institution and may perform confirming the supply completion quantity compared to the contract quantity, requesting drug delivery, requesting drug return, and reporting of transportation temperature excursion.

The study monitor type 120 may be a monitor agent monitoring the clinical trial and may request an order or return of clinical supplies to any institution. That is, the user of the study monitor type 120 may be a CRA (Clinical Research Associate) or CRC (Clinical Research Coordinator) belonging to the Korean Cancer Study Group, etc., and may perform confirming the supply completion quantity compared to the contract quantity, requesting drug delivery, requesting drug return, reporting of transportation temperature excursion, and inquiring about clinical information of various institutions.

The depot type 130 may perform storage or transportation of clinical drugs. That is, the user of depot type 130 may receive a drug delivery request and perform delivery.

The pharmaceutical general type 140 may conclude a contract for clinical trials such as Investigator Initiated Clinical Trials, and may request the user (administrator) of the pharmaceutical administrator type 150 to receive the contract. In addition, users of the pharmaceutical general type 140 may check the clinical drug supply status.

The pharmaceutical administrator type 150 may receive the requested contract and perform overall management of the preparation, transportation, and return of clinical trial supplies. Here, the user of the pharmaceutical administrator type 150 may have access to all functions provided by the clinical supplies management device (platform or system) according to the present disclosure.

According to an embodiment, the functions provided by the clinical supplies management method or apparatus of the present disclosure may be differentially provided according to the user types.

For example, for general users such as the study center type 110 and the study monitor type 120, a user registration function that can use the clinical supplies management device according to the present disclosure may be provided to the user themselves, and a confirmation function of contract fulfillment status may be provided, and a transportation/return request function and a confirmation function of receipt of the ordered supplies (drugs) may be provided. Here, general users such as the study center type 110 and the study monitor type 120 may not be provided with the function of registering a contract and managing changes to contract information, or a function of first approval of a general user account or study code authority, or a function of registering a transportation / return plan.

For example, for a user of the depot type 130, a user registration function may be provided based on the grant of an account of the user of the pharmaceutical administrator type 150, and may be provided with a transportation / return plan registration function and confirmation function of order / returned supplies(drugs). Here, the user of the Depot type 130 may not be provided with a transportation / return request function, function of registering a contract and managing changes to contract information, or a function of first approval of a general user account or study code authority. Here, the user of the depot type 130 may not be provided with a function to check the contract fulfillment status.

For example, for a user of the pharmaceutical general type 140, the user registration function may be provided based on the grant of an account of the user of the pharmaceutical administrator type 150, and may be provided with the function of registering a contract and managing changes to contract information, or a function of first approval of a general user account or study code authority. Here, the user of the pharmaceutical general type 140 may not be provided with a transportation / return request function, a function of registering a transportation / return plan, and a confirmation function of order / returned supplies(drugs).

For example, a user of the pharmaceutical administrator type 150 may be provided with all the functions provided by the clinical supplies management device (platform or system) of the present disclosure.

The above-described user registration function can be basically performed by selecting a My page menu (or an account setting menu such as user registration) by a user accessing a clinical supplies management device according to an embodiment of the present disclosure online, and first of all, when a general user requests user registration, a user of pharmaceutical general type or pharmaceutical administrator type approves it.

For example, a user of the study center type 110 and the study monitor type 120 can access the clinical supplies management device according to an embodiment of the present disclosure online, select one of the five user types shown in FIG. 1, enter user information, and select the participating clinical from the list of provided clinical trials (multiple selections are possible) to apply for membership. Here, the user information may include clinical drug receipt address information, and according to an embodiment, clinical drug receipt address information may be requested only for users of the study center type 110 and not for users of the study monitor type 120. Here, the clinical list provided may be a list of clinical trials filed by a user of the pharmaceutical general type 140 and approved by a user of the pharmaceutical administrator type 150.

Here, when a membership application is made from a user of the study center type 110 or the study monitor type 120, the clinical supplies management device according to an embodiment of the present disclosure may notify the user of the pharmaceutical general type 140 through an e-mail alarm that the application has been received. Here, the user of the pharmaceutical general type 140 may access the system (clinical supplies management device) to confirm the application for membership and request approval of the membership request to the user of the pharmaceutical administrator type 150, and here, an e-mail alarm may be sent to the user of the pharmaceutical administrator type 150. A user of the pharmaceutical administrator type 150 may access the system and approve a membership request, and if the membership request is approved, access to the user may be granted.

For example, a user of type depot 130 may receive a membership invitation e-mail generated by a user of the pharmaceutical administrator type 150 through the system. Here, the user of the pharmaceutical administrator type 150 may have negotiated in advance with the corresponding user of the depot type 130. A user of the depot type 130 who receives the invitation e-mail may perform a membership application by entering the user information required for membership registration, and the membership application may be approved by a user of the pharmaceutical general type 140 or the pharmaceutical administrator type 150. Here, the user information may include clinical and nation information awarded to the depot.

For example, a user of the pharmaceutical general type 140 may receive a membership invitation e-mail generated by the user of the pharmaceutical administrator type 150 through the system. Here, the user of the pharmaceutical administrator type 150 may have negotiated in advance with the corresponding user of the pharmaceutical general type 140. A user of the pharmaceutical general type 140 who receives the invitation e-mail may perform a membership application by entering the user information required for membership registration, and the membership application may be approved by a user of the pharmaceutical administrator type 150.

According to the embodiment, only users of the study center type 110 and the study monitor type 120 may be provided with a function of modifying information and requesting additional clinical access (secondary user registration request) after registering as a member. Here, when a secondary user registration request is received, an e-mail alarm may be sent to a user of the pharmaceutical general type 140, and a user of the pharmaceutical general type 140 may confirm the secondary user registration request, and may request to the user of the pharmaceutical administrator type 150 to approve the secondary user registration request. An e-mail alarm may be sent to a user of the pharmaceutical administrator type 150 in response to a secondary user registration request, and when a user of the pharmaceutical administrator type 150 requests approval of secondary user registration, the user may be granted access.

The user interface provided to the user using the clinical supplies management apparatus according to an embodiment of the present disclosure may include a dashboard interface, and at least some of the sections constituting the dashboard interface may be exposed differently for each user type shown in FIG. 1.

For example, the dashboard interface may include contract fulfillment status, inventory status, transportation status, return status, new contract reception, and new account approval sections.

For example, the contract fulfillment status section may be provided through the supply consumption status sharing interface, and may be a section showing the quantity of supplies (drugs) released or the quantity supplied to the study center type user compared to the contract quantity aggregated in the contract application tab. Here, the contract fulfillment status section may be provided to all types of users except the depot type 130. That is, the contract fulfillment status section may be provided to users of the study center type 110, the study monitor type 120, the pharmaceutical general type 140, and the pharmaceutical administrator type 150. Here if there is supply requested for return, the contract quantity displayed in the contract fulfillment status section may be updated based on the approval of the user of the pharmaceutical administrator type 150 for the return request.

For example, the inventory status section may be a section indicating the quantity of supplies (drugs) by location or condition. Here, the inventory status section may be provided only to users of the depot type 130 and the pharmaceutical administrator type 150. Here, the inventory status section may display the available inventory amount for each clinical trial, and information on drugs with an expiration date of less than 3 months may be highlighted.

For example, the transportation status section may be a section indicating information on supplies for which a transportation request (providing request) has been made but has not been processed for transportation completion. Here, the transportation status section can be provided to all types of users. However, the user corresponding to the study monitor type 120 may be provided with an entire list of orders for supplies of all users of the study center type 110 participating in the clinical trial, and the user corresponding to the study center type 110 may be provided with information on the transportation of the supplies ordered from the study center.

For example, the return status section may be a section indicating information on items for which a return request has been made but has not been processed return completed. Here, the return status section may be provided to all types of users. However, the user corresponding to the study monitor type 120 may be provided with an entire list of returned supplies of all users of study center types 110 participating in the clinical trial, and the user corresponding to the study center type 110 may be provided with information on the return of the supplies ordered from the study center.

For example, the new contract reception section may be a section indicating items that have newly received a contract related to a clinical trial but have not been approved by a user of the pharmaceutical administrator type 150. Here, the new contract reception section may be provided to all types of users except the depot type 130. That is, the new contract reception section may be provided only to users of the study center type 110, the study monitor type 120, the pharmaceutical general type 140, and the pharmaceutical administrator type 150.

For example, the new account approval section may be a section indicating items that have been newly requested through membership registration or modification of my account, but have not yet been approved by a user of the pharmaceutical administrator type 150. Here, the new account approval section may be provided only to users of the pharmaceutical general type 140 and the pharmaceutical administrator type 150.

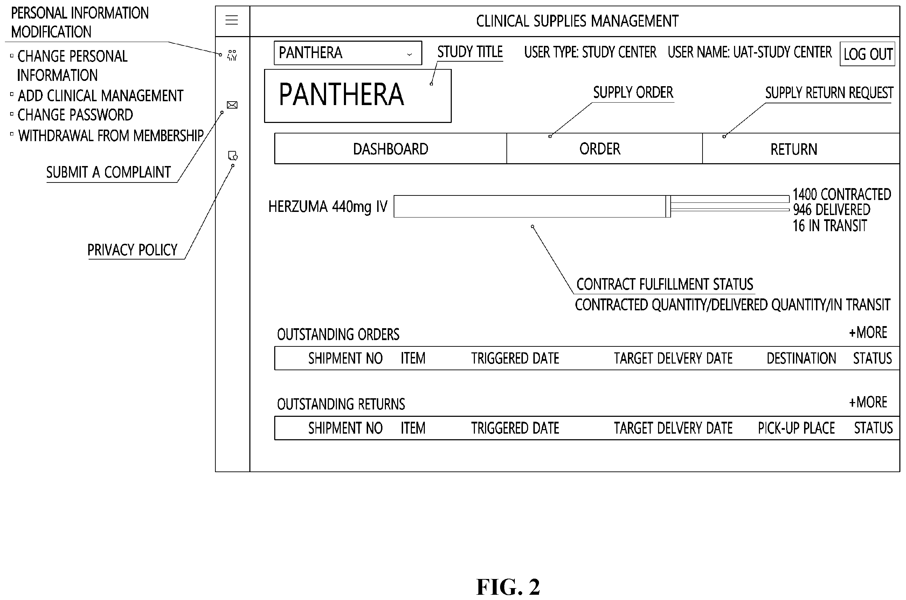

FIG. 2 illustrates an example of a user page provided to a user of a study center type or a study monitor type.

Referring to FIG. 2, it may be seen that the user page includes a dashboard interface.

In particular, the dashboard interface may display a contract fulfillment status section, and at this time, the contract fulfillment status section may be provided through a supply consumption status sharing interface for sharing the consumption status of the entire supplies allocated to the clinical trial to participants involved in the clinical trial. Here, the supply consumption status sharing interface includes graphs representing the total cumulative order quantities of the entire participants involved in the clinical trial(Contracted), the total cumulative delivery completion quantities of the entire participants(Delivered), and the total delivery quantities in progress of the entire participants(In Transit) as shown in FIG. 2. That is, the total cumulative order quantities(Contracted) may be the number of supplies that the pharmaceutical company has agreed to supply, and the pharmaceutical company may not be able to supply the quantity in excess of the contracted quantity. Here, the total cumulative delivery completion quantities of the entire participants(Delivered) may be the quantity delivered to all study centers, and the total delivery quantities in progress of the entire participants(In Transit) may be the quantity currently delivered to all study centers.

In particular, unlike pharmaceutical companies or sponsor initiated clinical trial, it is very important for investigator-initiated clinical trials to share the supply consumption status of all clinical supplies allocated to the clinical trial to the clinical trial participants through the supply consumption status sharing interface. That is, the clinical supplies management apparatus and method according to an embodiment of the present disclosure allow various institutions participating in the clinical trial not only to know the progress of their orders, but also to grasp the supply consumption status of the total volume allocated to the clinical trial (consumption status of the total cumulative orders of the clinical participating institutions). Here, the clinical trial requester corresponding to the investigator-initiated clinical trial may be each research institution (such as a professor at a university hospital).

As shown in FIG. 2, the user page may include a supply order tab(Order) and a return request tab (Return) in addition to the dashboard tab, and may include outstanding orders and outstanding returns item.

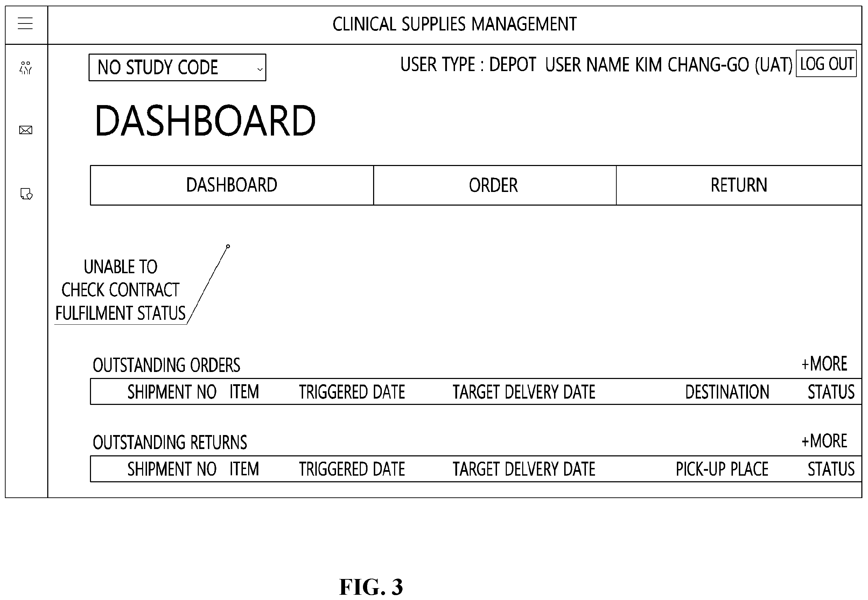

FIG. 3 illustrates an example of a user page provided to a user of the depot type.

Referring to FIG. 3, it may be seen that the user page includes a dashboard interface.

In particular, the dashboard interface provided to users of the depot type may not display contract fulfillment status section. That is, other types of users may see the contract fulfillment status section, but the depot type user may not see the contract fulfillment status section.

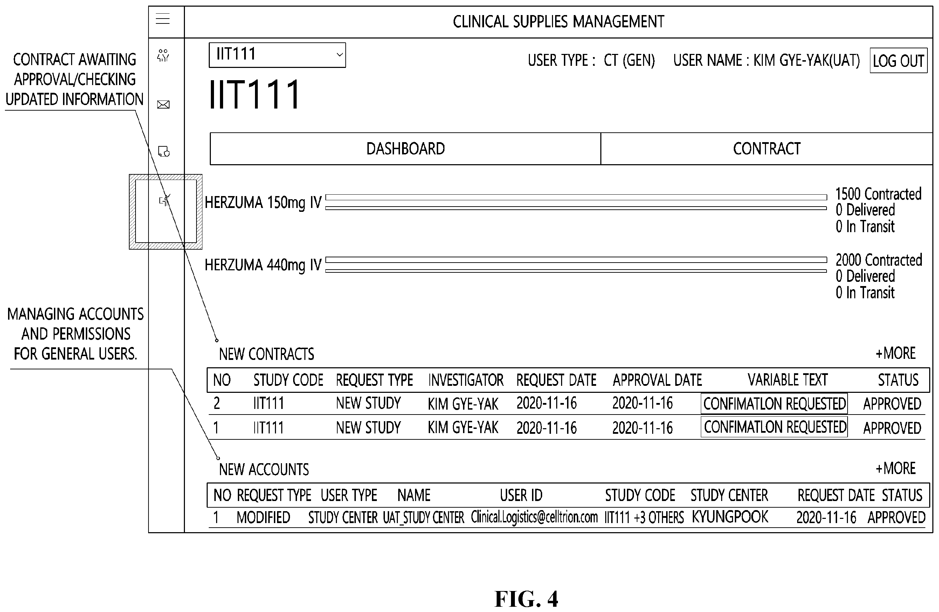

FIG. 4 illustrates an example of a user page provided to a user of the pharmaceutical general type.

Referring to FIG. 4, it may be seen that the user page includes a dashboard interface.

In particular, the dashboard interface provided to users of the pharmaceutical general type displays contract fulfillment status section, and here, the contract fulfillment status of all supplies (drugs) related to the clinical trial can be displayed. That is, the contract fulfillment status section may be provided through a supply consumption status sharing interface for sharing the consumption status of the entire supplies allocated to the clinical trial to participants involved in the clinical trial.

In addition, the dashboard interface provided to users of the pharmaceutical general type may include new contract reception section and new account approval sections. Here, the new contract reception section may be for confirmation of the contract pending approval and the changes to the contract. Here, the new account approval section may be for account and authority management such as a general user.

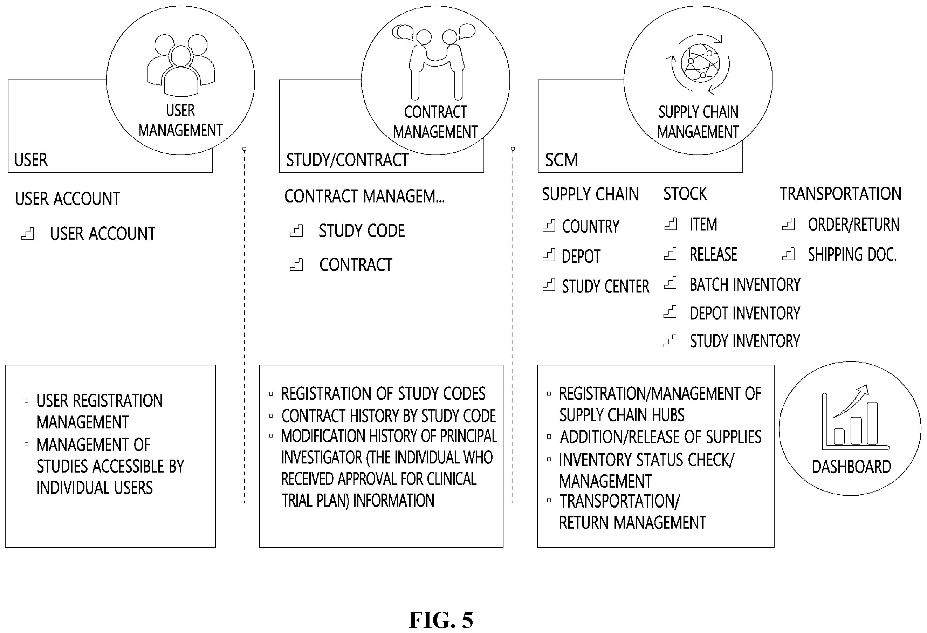

FIG. 5 illustrates an example of a user page provided to a user of the pharmaceutical administrator type.

Referring to FIG. 5, it may be seen that the user page provides various interfaces such as a membership management interface, a contract management interface, and a supply chain management (SCM) interface.

In particular, through the member management interface, functions such as new membership registration management of users or study management accessible by member may be provided. Here, functions such as registration of study codes, confirmation of contract conclusion details for each study code, and information management of persons (principal investigators) who received approval for clinical trial plan may be provided through the contract management interface. Here, through the supply chain management interface, functions such as registration / management of supply chain (depot) base, addition / removal of supplies, confirmation / management of improvement status, and transportation / return management may be provided. As shown in the example shown in FIG. 5, a dashboard interface may also be provided to a user of the pharmaceutical administrator type, and in particular, the dashboard interface provided to the user of the pharmaceutical administrator type may include the above-described contract fulfillment status, inventory status, transportation status, return status, new contract reception, and new account approval sections.

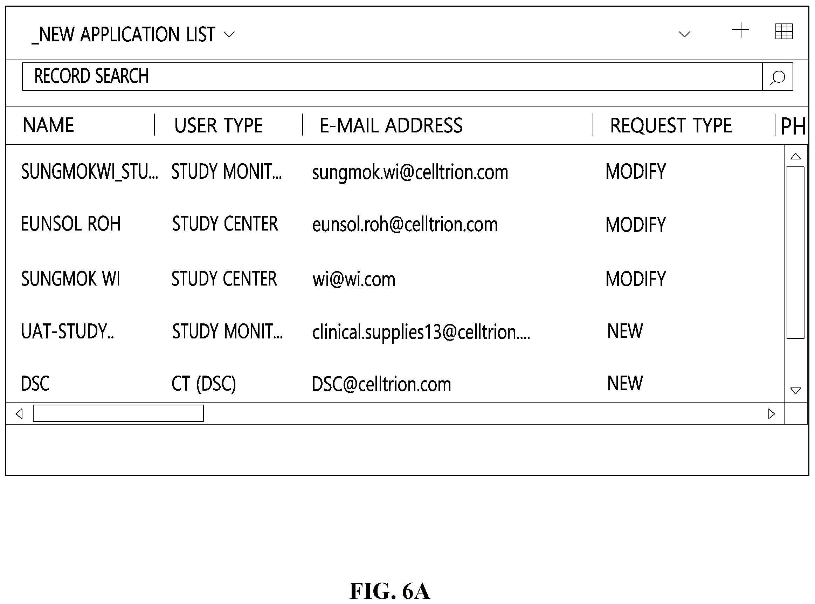

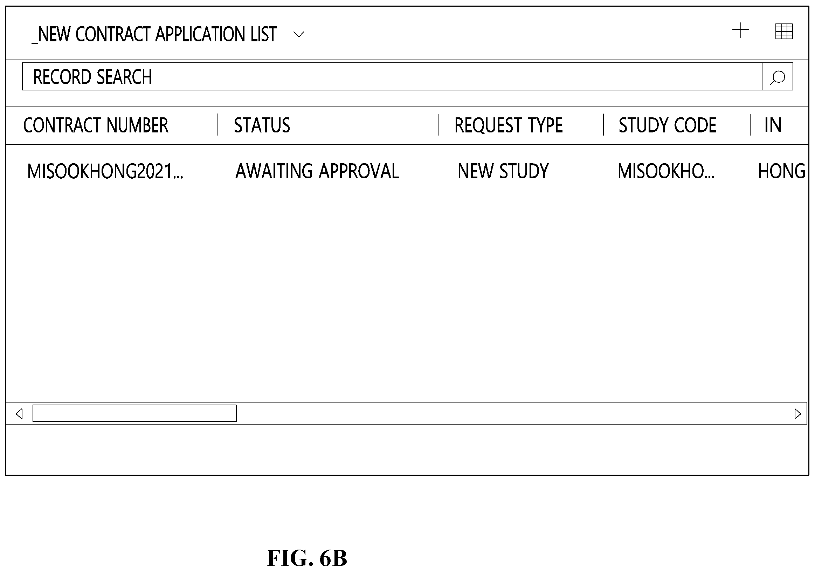

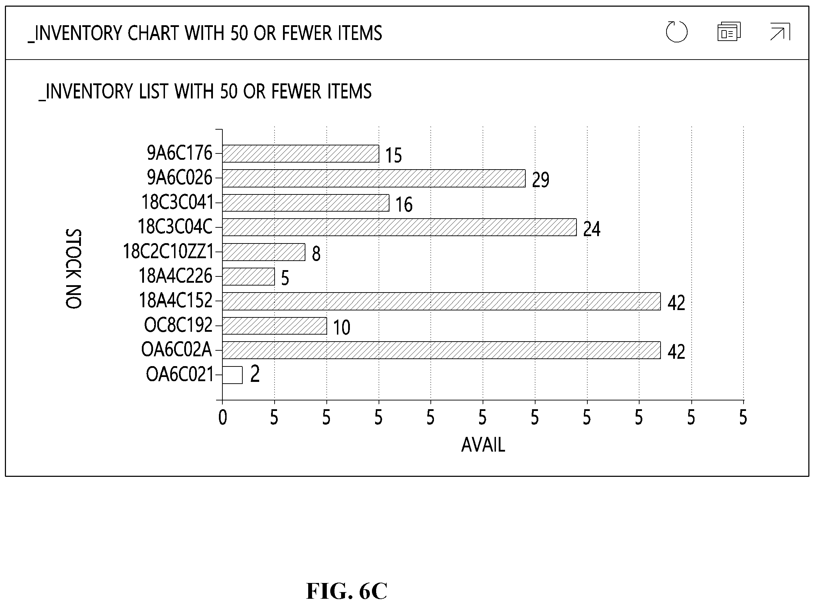

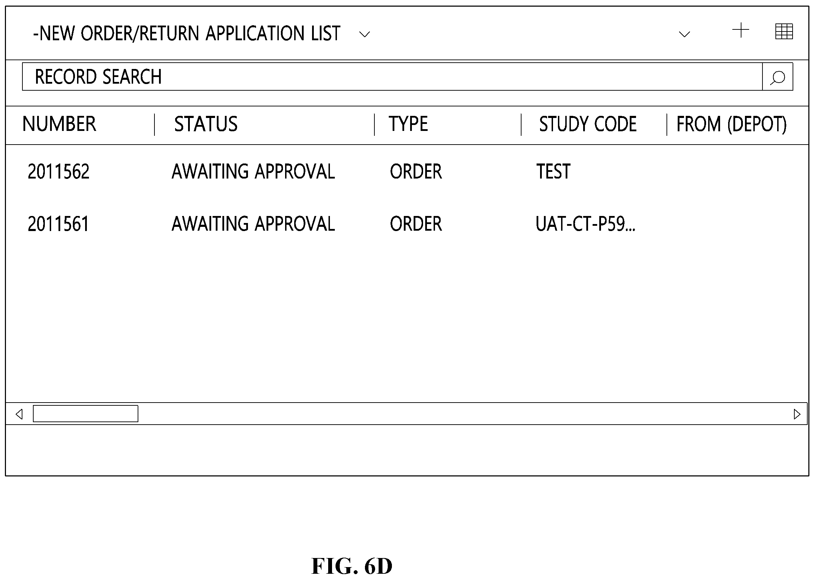

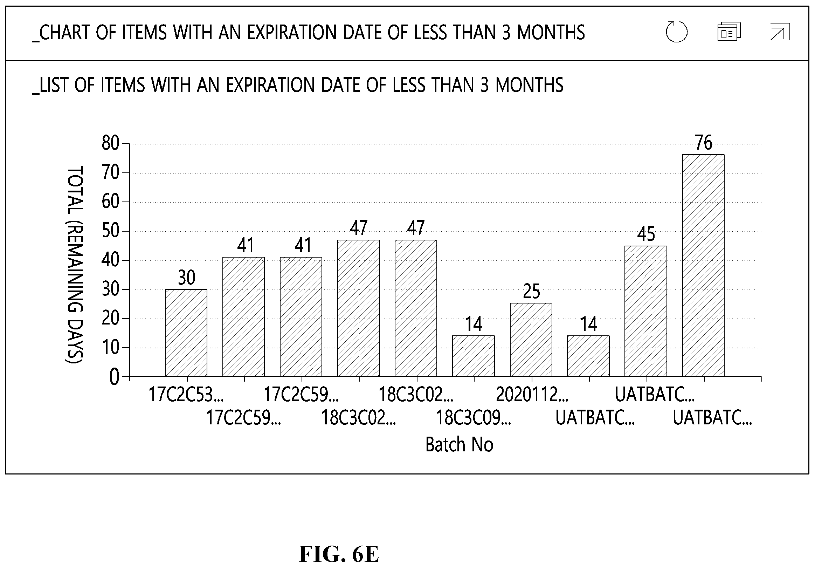

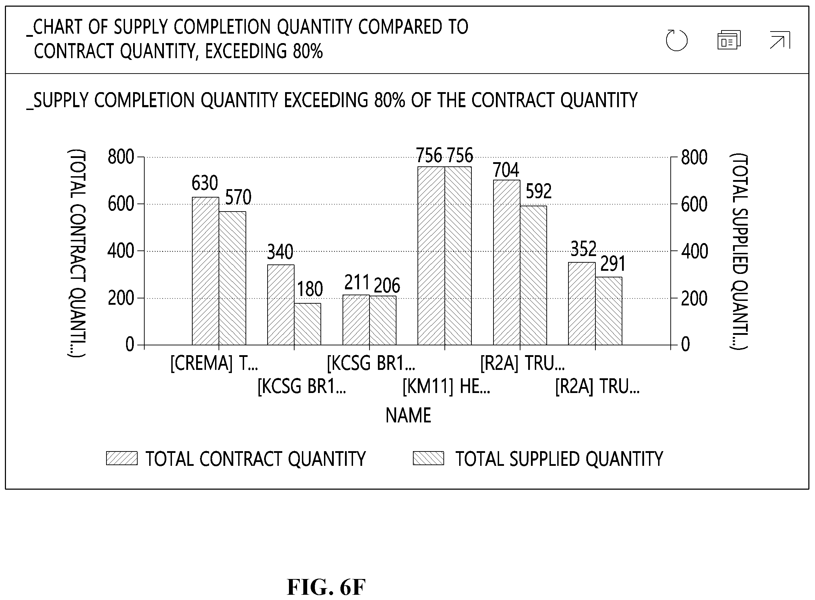

FIGS. 6a to 6f illustrates other examples of a user page provided to a user of the pharmaceutical administrator type.

Referring to FIGS. 6a to 6f, the user of the pharmaceutical administrator type may be provided with a new application list page, a new contract application list page, a chart page of 50 or fewer inventory, a new order / return application list page, an item list page with an expiration date of less than 3 months, and a chart page of 80% or more of the supplied quantity compared to the contract quantity.

On the new application list page, it may be seen that the details of the request for new membership registration or account grant. Here, the displayed details may include information such as a user name, user type, e-mail address, request type, phone number, etc., and in particular, the request type may be either new or modified.

The new contract application list page may display a list of contracts related to the newly filed clinical trial. Here, the displayed details may include the contract number, status, request type, test code, and researcher information.

On the chart page of 50 or fewer inventory, items with an inventory of 50 or less can be displayed as charts. The reference quantity (e.g., 50) displayed according to the embodiment may be changed.

The new order / return application list page may display orders or return requests that have not yet been processed after the application. Here, the displayed details may include information such as serial number, status, type (order or return), test code, etc.

On the item list page with an expiration date of less than 3 months, items with an expiration date of less than 3 months of the item (drug) may be displayed as a chart. The reference period (e.g., 3 months) displayed according to the embodiment may be changed.

On the chart page of 80% or more of the supplied quantity compared to the contract quantity, items with a performance rate (supply completion quantity compared to the contract quantity) of 80% or more may be displayed as a chart. The reference ratio (e.g., 80%) displayed according to the embodiment may be changed.

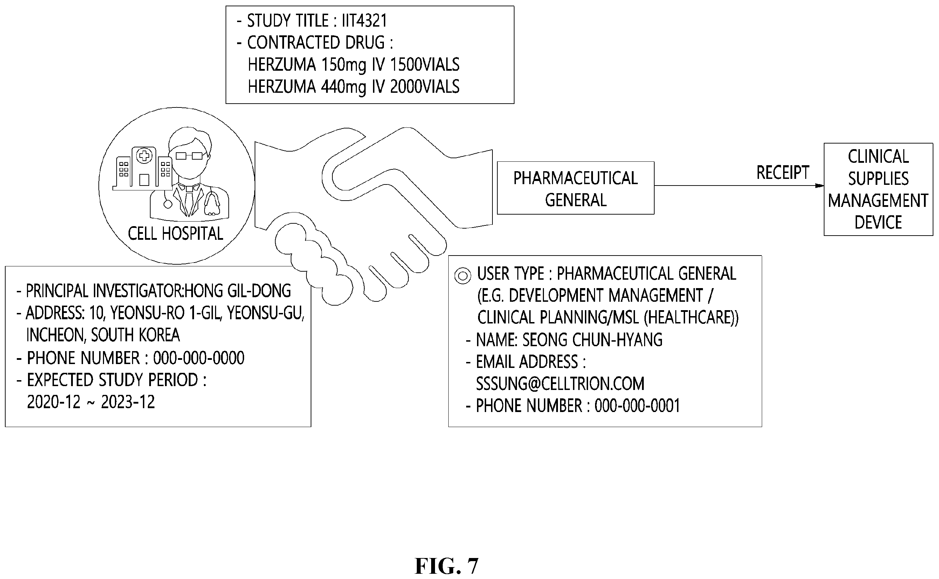

FIG. 7 illustrates an example of signing a contract related to a clinical trial.

Referring to FIG. 7, it can be seen that the study center type user 'Hong Gil-dong' and the pharmaceutical general type user 'Sung Chun-hyang' participate in the contract related to the clinical trial. That is, the contents of the contract negotiated by the study center type user 'Hong Gil-dong' and the pharmaceutical general type user 'Sung Chun-hyang' are entered through a clinical supplies management device according to an embodiment of the present disclosure, and the article (drug) management corresponding to the contract content can be performed. Here, the contract may include the research name (IIT4321) and the contract drug information (Herzuma 150mg IV 1500Vials, Herzuma 440mg IV 2000Vials).

The interface for receiving and managing contract contents can only be displayed to users of the pharmaceutical general and users of the pharmaceutical administrator type.

For example, when a user of the pharmaceutical general type inputs information related to a new contract into a clinical supplies management device according to an embodiment of the present disclosure, an e-mail alarm is sent to a user of the pharmaceutical administrator type, and when a user of the pharmaceutical administrator type confirms the contents of the new contract, an e-mail alarm is sent to the user of the pharmaceutical general type, and the contract contents can be received and managed in the order in which the user of the pharmaceutical general type of enters additional information (protocol, contract, variable text, etc.) related to the contract content. According to the embodiment, an e-mail alarm may be sent if the user of the pharmaceutical general type does not finally complete the contract receipt processing even after the predetermined period has elapsed after entering additional information related to the contract content.

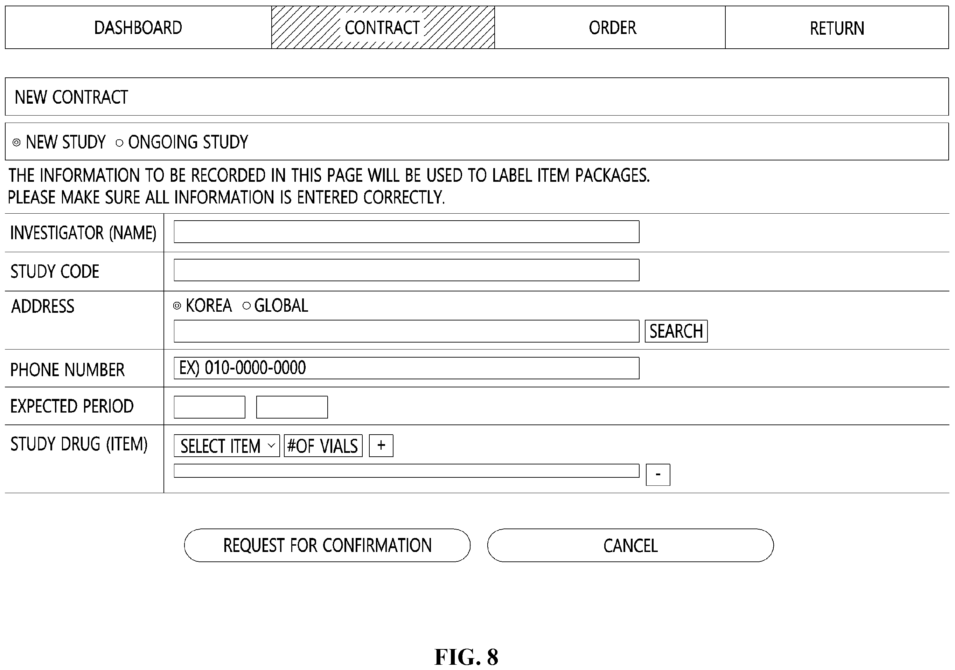

FIG. 8 illustrates an example of an interface screen provided to the user of the pharmaceutical general type for applying a contract.

Referring to FIG. 8, a user of the pharmaceutical general type may activate the Contract tab of the provided interface to enter information related to the contents of the contract (investigator, trial code, address, telephone number, estimated period, test drug) and request confirmation from a user of the pharmaceutical administrator type. Here, the information related to the contract may include whether it is a new clinical trial contract (New Study) or a modification / additional contract (Ongoing Study) of the existing contract, and the information of the person (principal investigator ) who has received the approval of the clinical trial plan may be included. In particular, the information of the person who has received the approval of the clinical trial plan is information included in the variable text to be printed on the label of the product (drug), and may be entered through an input interface in which a cautionary phrase requesting accurate input is displayed. For example, when the information related to the contract is entered, an alarm email may be sent to a user of the pharmaceutical general type and the user of the pharmaceutical administrator type.

A user of the pharmaceutical administrator type who receives the alarm email may check and approve the entered contract contents, and only the clinical trials approved by the user of the pharmaceutical administrator type can be activated for use in the clinical supplies management device according to an embodiment of the present disclosure.

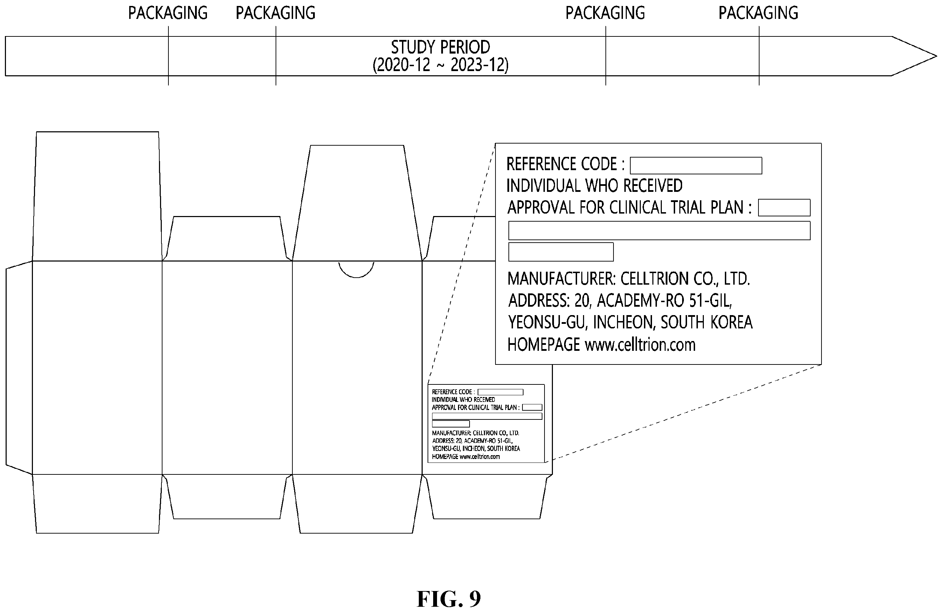

FIG. 9 illustrates an example of variable text.

Referring to FIG. 9, it can be seen that the variable text is printed on the product (drug) package and includes the information of the person (principal investigator ) who has received clinical trial plan approval.

Confirmation of variable text information may be requested from a user of the pharmaceutical administrator type to a user of the pharmaceutical general type, and the confirmation request may be information of a person who has received approval of the clinical trial plan. According to the embodiment, the confirmation request of the variable text information may require the input of a confirmation due date, and based on the confirmation request of the variable text information, additional information to be printed on the product packaging may be provided in addition to the information of the person who has received the approval of the clinical trial plan.

Here, the variable text information may be information that must be printed at the time of packaging of the item (drug), and the confirmation of the variable text information is a very important procedure for the supply manager of the clinical drug (user of the pharmaceutical administrator type) to confirm the information that must be confirmed before the time of drug packaging. At this time, the contractor who is requested to confirm can check the validity of the variable text information by clicking a simple confirmation button in the system (clinical supplies management device).

When the confirmation of the variable text information is completed by the user of the pharmaceutical general type, an alarm email may be sent to the user of the pharmaceutical general type and the user of pharmaceutical administrator type, and finally the user pharmaceutical administrator type may finally confirm the variable text information. Here, the confirmation request date for requesting confirmation of variable text information from a user of the pharmaceutical general type and the confirmation date for the confirmation of the variable text information by the user of the pharmaceutical general type may be recorded in a clinical supplies management device according to an embodiment of the present disclosure.

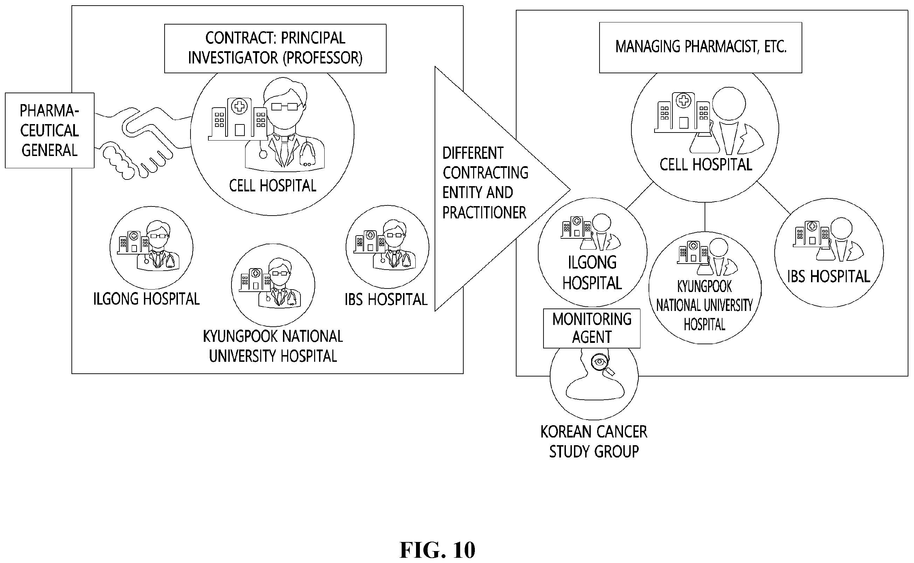

FIG. 10 illustrates the situation that is different between the contracting entity and the practitioner of the contract related to the clinical trial.

Referring to FIG. 10, the contracting entity of the clinical trial center may be the principal investigator (professor), but the entity who actually conducts the clinical trial may be another user of the trial center or a user of another institution in cooperation with the clinical trial center.

In consideration of such a situation, the clinical supplies management apparatus and method according to an embodiment of the present disclosure allow users of the study center type or study monitor type to register other users (secondary users) in the system, thereby improving the convenience of clinical trial participants. That is, the clinical supplies management apparatus and method according to an embodiment of the present disclosure may request new user registration by a general user of a study center type or a study monitor type without the support of an IT developer.

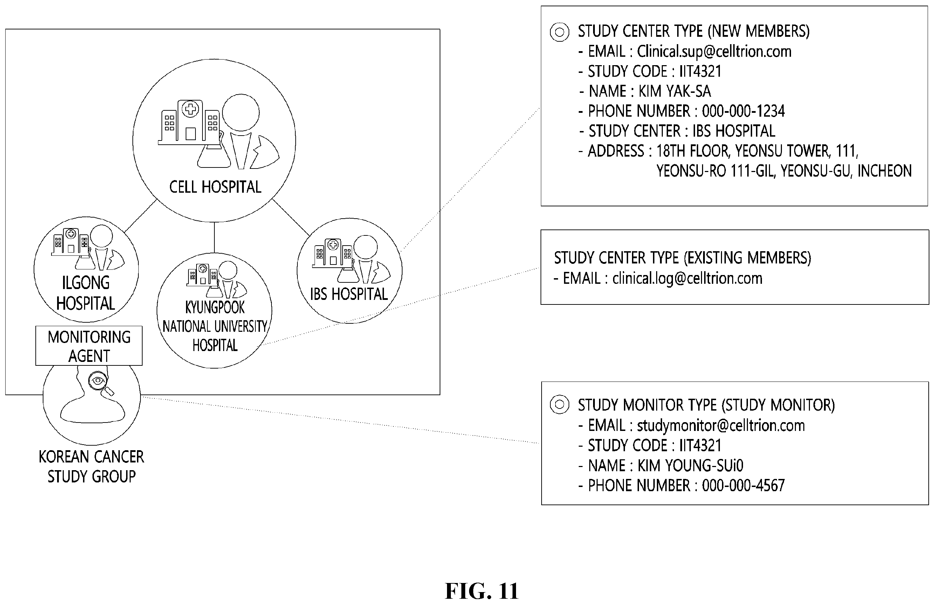

FIG. 11 illustrates an example of user membership registration of a user corresponding to a practitioner.

Referring to FIG. 11, it can be seen that a new member of the study center type or a new member of the study monitor type may be added corresponding to the clinical trial registered in the system. Here, not only new members but also users corresponding to existing member may be additionally registered corresponding to the clinical trial.

Furthermore, the clinical supplies management apparatus and method according to an embodiment of the present disclosure can be set up such as new clinical trials, user authorities, and supply chain hubs without the support of IT developers, and new clinical trials and user permission functions are provided in the system itself.

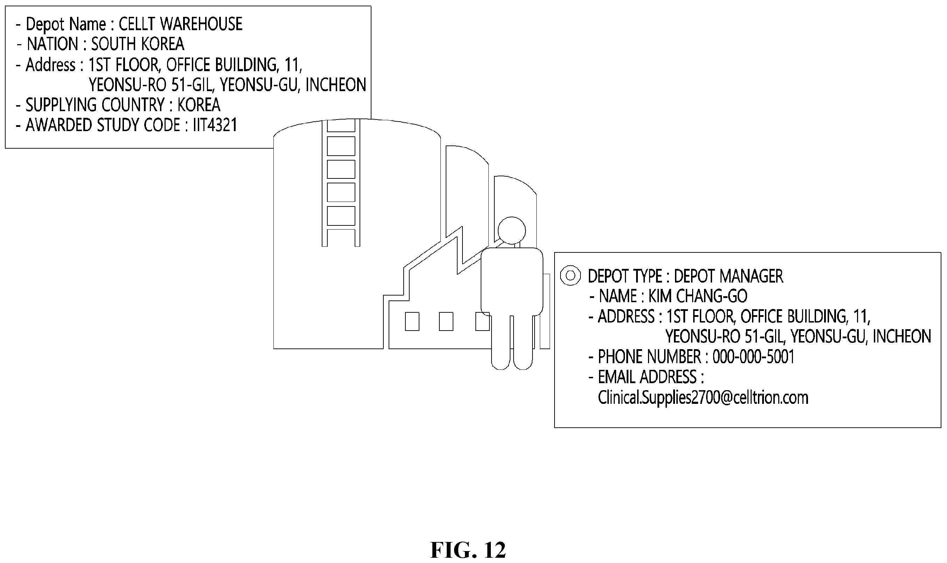

FIG. 12 illustrates an example of the depot type user registration.

Referring to FIG. 12, it can be seen that the hub manager of a supply chain hub called a 'celt warehouse' is registered in the system as a depot type user.

Registration of a new depot type user may be performed by a user of the pharmaceutical administrator type, and the information required for registration of a new depot type user may include the depot name, address, awarded clinical information, and country information covered. Here, the awarded clinical information may be entered by selecting one of the list of clinical trials registered in the system.

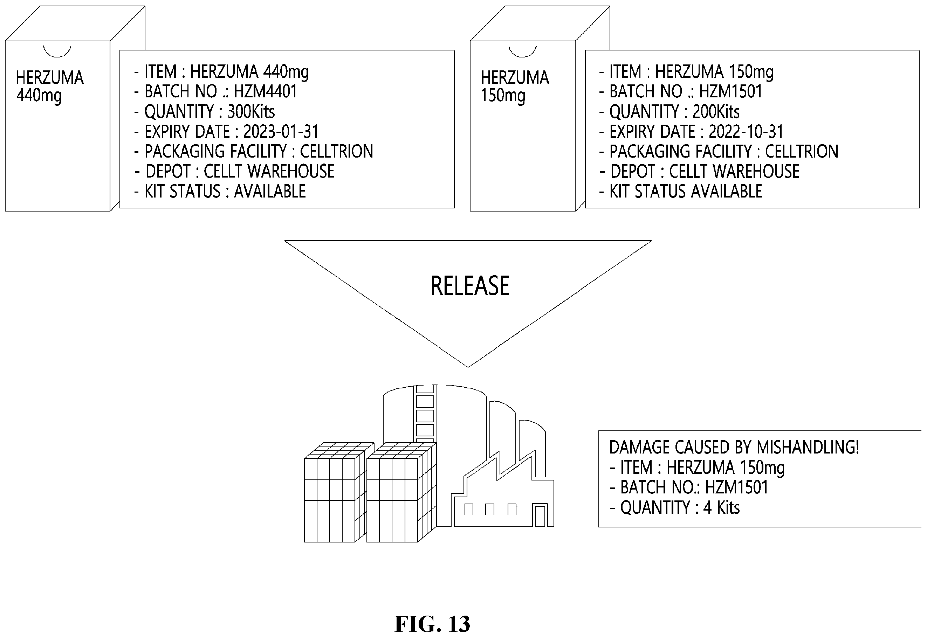

FIG. 13 illustrates an example in which a packaged drug is released.

Referring to FIG. 13, it can be seen that two drugs, "Herzuma 440mg" and "Herzuma 150mg", are released to a supply chain hub called a 'celt warehouse'. Here, the release information provided for the release of the drug may include a study code, an item, a batch number, an expiry date, a release quantity, a packaging facility, an initial location, a depot, and kit status information.

The test code may be determined by the user's selection of the list of test codes received in the system.

The item may be determined by the user's selection of the contract drug list received in the system.

The batch number can be entered directly from a user of the pharmaceutical administrator type, etc.

The expiration date can be entered in the YYYY-MM-DD format.

The release quantity may be the quantity of the items (drugs) being distributed, and the packaging facility may be the institution that packaged the items (drugs).

The initial location may be determined by the user's selection of the depot list registered in the system.

The kit status information may indicate one of the thirteen states defined in Table 1 below.

**[Table 1]**

| Status Name | Status Description |
|---|---|
| Available | Available inventory, deduction from available quantity at the time of receipt of the order |
| In Transit | Order has been received but has not yet arrived at the study center. |
| Quarantined | A problem with the drug was found and is under investigation |
| Damaged | The drug is damaged |
| Expired | The drug is expired |
| Repackaging | Repackaging for other studies |

| Others | Other status |
|---|---|
| To be destroyed (non-temp controlled) | It was never distributed to the study center and was in constant storage at depot, but it is certain that it will not be used. |
| Destroyed | The drug is destroyed |
| Received at Study Center | Arrived at the study center with no defect |
| Others at Study Center | Arrived at the study center, but found to be defective, and the quantity not counted as the supply quantity |
| Returned | Returned from the study center and refrigerated at 2-8°C |
| Returned (non-temp controlled) | Returned from the study center and kept at room temperature |

After the release information is entered, an alarm mail may be sent to the user of the pharmaceutical administrator type.

In particular, the kit status information in the release information may be updated automatically or manually after the release. For example, when an order or return of an item (drug) occurs, the kit status information may be updated according to the situation, and inventory deduction or increase processing may be performed. For example, an expired drug may automatically change its status to Expired.

Manual update of kit status information can only be performed by a user of the pharmaceutical administrator type. Here, in order to update the kit status information, a test code, a moving quantity, a reason for movement, and updated status information may be entered. Here, the test code may be entered based on the user's selection of the activated test code list. Here, the updated state may be any one of the states described in Table 1 above. Here, when a manual update of the kit status information is performed, an alarm email may be sent to a relevant user of the depot type and a user of the pharmaceutical administrator type.

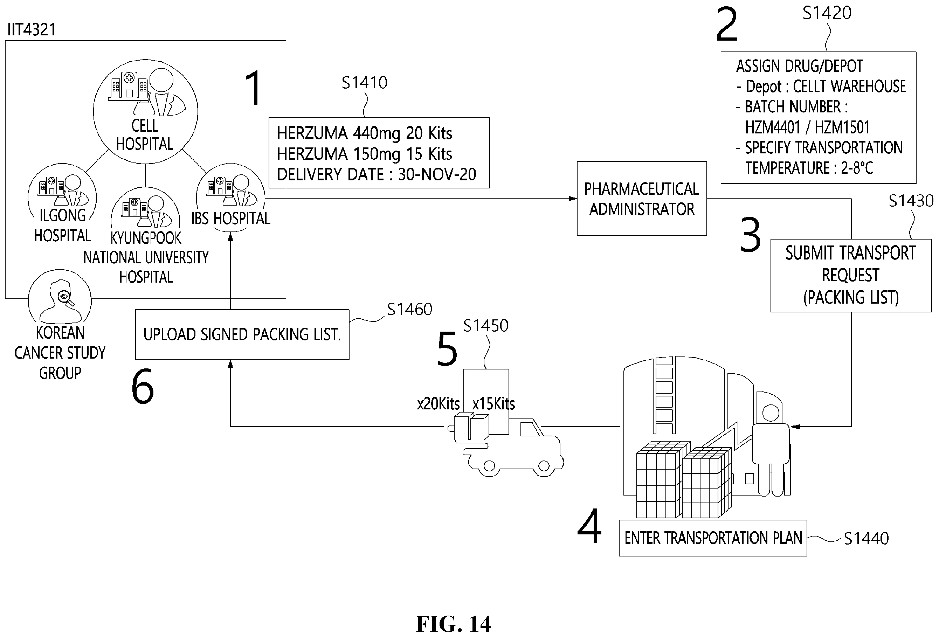

FIG. 14 illustrates an example of supplying items using a clinical supplies management apparatus according to an embodiment of the present disclosure.

Referring to FIG. 14, it can be seen that a request for supply of items (order for items) is made by a user of a study center type or a study monitor type. According to an embodiment, a user of the depot type or a user of the pharmaceutical administrator type may also be able to request the supply of items. For example, a request for a shipment to be shipped to a study center may be made by a user of the study center type, the study monitor type, or the pharmaceutical administrator type. For example, a request for supply for a shipment to be shipped to a depot may be made by a user of the pharmaceutical administrator type.

First, an order for items may be created by a user of a study center type, a study monitor type, or a pharmaceutical administrator type at step S1410.

Here, the clinical supplies management apparatus according to an embodiment of the present disclosure may receive an item order through an interface indicating information to order at least N (natural number) business days before the date of needing the item.

Here, requestor information, recipient information, item order information (item, dosage, order quantity, etc.), target delivery date information, and the like may be entered to create an item order.

When an item order is generated, a clinical supplies management device according to an embodiment of the present disclosure may assign the article at step S1420.

Here, a shipping number corresponding to the item order may be assigned according to a predetermined logic.

Here, the item (drug) allocation determines the study corresponding to the ordered item, the depot allocated to the clinical trial is assigned, and the product condition is available among the corresponding product inventory of the depot.

Here, if the ordered items are not sufficient for the assigned depot, a notification is sent to a user of the pharmaceutical administrator type, and a manual order may be generated by the user of the pharmaceutical administrator type who receives it. Here, the items remaining within one month of the expiration date may be excluded from the allocation.

When the allocation of items is completed, the clinical supplies management device according to an embodiment of the present disclosure may send an alarm email to a user of the pharmaceutical administrator type.

A user of the pharmaceutical administrator type who receives the alarm e-mail can check the status of the allocation of the items in the system and approve the transportation if there is no abnormality. Here, a user of the pharmaceutical administrator type can assign the item allocation as a manual if he wants to replace the batch.

The contents of the transportation authorization for the shipment approved by the user of the pharmaceutical administrator type may be sent to the user of the depot type in the form of a shipping request or packing list at step S 1430.

Here, the transportation approval may be sent by e-mail, and a Uniform Resource Locator (URL) of a web page for entering the transportation plan may be added to the sent e-mail.

A user of the depot type who has received the transportation approval information may enter the transportation plan through a web page provided by a clinical supplies management device according to an embodiment of the present disclosure at S1440.

Here, the input of the transportation plan may be performed not only by a user of the depot type but also by a user of the pharmaceutical administrator type.

Here, the transportation plan may include ETD (Estimated Time of Despatch) information, ETA (Estimated Time of Arrival) information, courier name, and tracking number.

Here, if the transportation plan is not entered within a predetermined period after step S1430, the clinical supplies management device according to an embodiment of the present disclosure may send an alarm email to a user of the depot type and a user of the pharmaceutical administrator type.

When the input of the transportation plan is completed at step S1440, an alarm email may be sent to a user of the study center type or study monitor type corresponding to the requestor or recipient, the user of the depot type, and the user of the pharmaceutical administrator type. Here, the alarm email may include not only transportation plan related contents such as ETD, ETA, and batch information, but also confirmation of the completion of order receipt, and may include POD (Proof of Delivery) form information. Here, the POD format corresponding to the POD format information may include a thermometer start / end time information input box. Here, the alarm email may include URL information for entering transportation completion information (supply completion information) along with a request message to enter the POD and temperature record when transportation is completed. According to the embodiment, the alarm email may include a warning message not to use the item for the safety of the patient in the event of a problem such as damage to the transported item.

The transportation of items (drugs) is carried out at step S1450 corresponding to the entry of the transportation plan at step S1440.

When the items arrive at the study center where the items are ordered, the user of the study center type, the study monitor type, or the pharmaceutical administrator type enters the transportation completion information at step S1460. Here, the user of the study center type or the study monitor type inputting the transportation completion information may be a user corresponding to the requestor or the recipient. According to an embodiment, a user of the depot type may also be able to input transportation completion information.

That is, the study center performs a confirmation of the completion of the transport to the system (clinical supplies management device) when the items (drugs) arrive. Here, the transportation completion information input corresponding to the confirmation of the completion of the transportation case may include POD (Proof of Delivery) information, item damage information, and temperature record information. Here, the POD information may be an electronic document prepared in a standardized form, and the item damage information may include whether the transported item is damaged. The temperature record information may include temperature excursion and temperature data information. At this time, the temperature data information may be registered as a file.

If there is a problem with the transported items, it may be determined whether to count the supply quantity by consultation between the user of the study center type or study monitor type and the user of the pharmaceutical administrator type for the defective items. Here, a user of the pharmaceutical administrator type can change the status to the aforementioned "Others at Study Center" in the quantity corresponding to the defective items. Here, items in the "Others at Study Center" status may not be counted in quantities supplied to the study center.

When the transportation completion information (supply completion information) is entered without any problems, the transportation process is terminated after updating the information on the completed shipment.

If the transportation completion information is not uploaded even after the ETA included in the transportation plan entered at step S 1440 has passed, an alarm email may be sent to the user of the study center type, the study monitor type, the depot type, and / or the pharmaceutical administrator type.

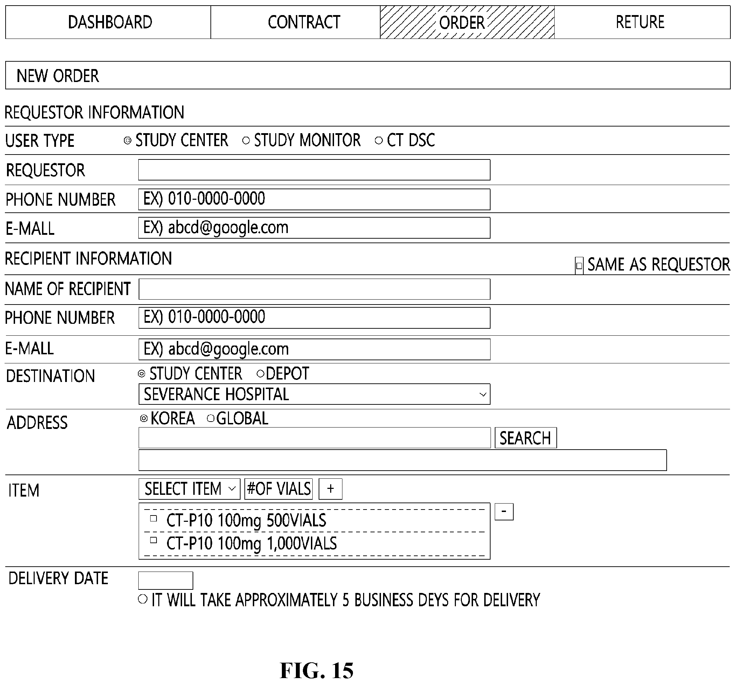

FIG. 15 is an example of an interface page provided to a user of a study center type for ordering items.

Referring to FIG. 15, a user of the study center type may activate the Order tab of the provided interface to enter the product order creation information for requesting the supply of an item (drug) related to his or her clinical trial.

Supplies of items from one depot to another may also be provided.

Here, a request for the supply of items (order for items) for the supply of items from one depot to another depot may be performed by a user of the pharmaceutical administrator type.

Here, requestor information, recipient information, product order information (items, dosage, order quantity, etc.), target delivery date information, and transportation temperature maintenance information may be entered for the creation of an item order. Here, the requester information may be selected from the user list of the pharmaceutical administrator type registered in the system. Here, the recipient information may be selected from the list of users of the depot type registered in the system.

When an order for items from depot to depot is created, the system can assign transportation number and assign items (drugs) according to a predetermined logic. Here, the generated order may be sent to depot in the form of a shipping request.

Upon receiving the shipment request, the corresponding user of the depot may enter the shipping plan for the newly created order.

Here, the input of the transportation plan may be performed not only by a user of the depot type but also by a user of the pharmaceutical administrator type.

Here, the transportation plan may include ETD (Estimated Time of Despatch) information, ETA (Estimated Time of Arrival) information, courier name, and tracking number.

Here, the transportation plan may include ETD (Estimated Time of Despatch) information, ETA (Estimated Time of Arrival) information, courier name, and tracking number.

Here, if the transportation plan is not entered within a predetermined period after the order creation, the clinical supplies management device according to an embodiment of the present disclosure may send an alarm email to a user of the depot type and a user of the pharmaceutical administrator type.

When the input of the transportation plan is completed, an alarm email may be sent to a user of the study center type or study monitor type corresponding to the requestor or recipient, the user of the depot type, and the user of the pharmaceutical administrator type. Here, the alarm email may include not only transportation plan related contents such as ETD, ETA, and batch information, but also confirmation of the completion of order receipt, and may include POD (Proof of Delivery) form information. Here, the POD format corresponding to the POD format information may include a thermometer start / end time information input box. Here, the alarm email may include URL information for entering transportation completion information (supply completion information) along with a request message to enter the POD and temperature record when transportation is completed. According to the embodiment, the alarm email may include a warning message not to use the item for the safety of the patient in the event of a problem such as damage to the transported item.

The transportation of items (drugs) is carried out corresponding to the entry of the transportation plan.

That is, the study center performs a confirmation of the completion of the transport to the system (clinical supplies management device) when the items (drugs) arrive. Here, the transportation completion information input corresponding to the confirmation of the completion of the transportation case may include POD (Proof of Delivery) information, item damage information, and temperature record information. Here, the POD information may be an electronic document prepared in a standardized form, and the item damage information may include whether the transported item is damaged. The temperature record information may include temperature excursion and temperature data information. At this time, the temperature data information may be registered as a file.

If there is a problem with the transported items, it may be determined whether to count the supply quantity by consultation between the user of the study center type or study monitor type and the user of the pharmaceutical administrator type for the defective items. Here, the status update of the item may be performed by a user of the pharmaceutical administrator type.

When the transportation completion information (supply completion information) is entered without any problems, the transportation process is terminated after updating the information on the completed shipment.

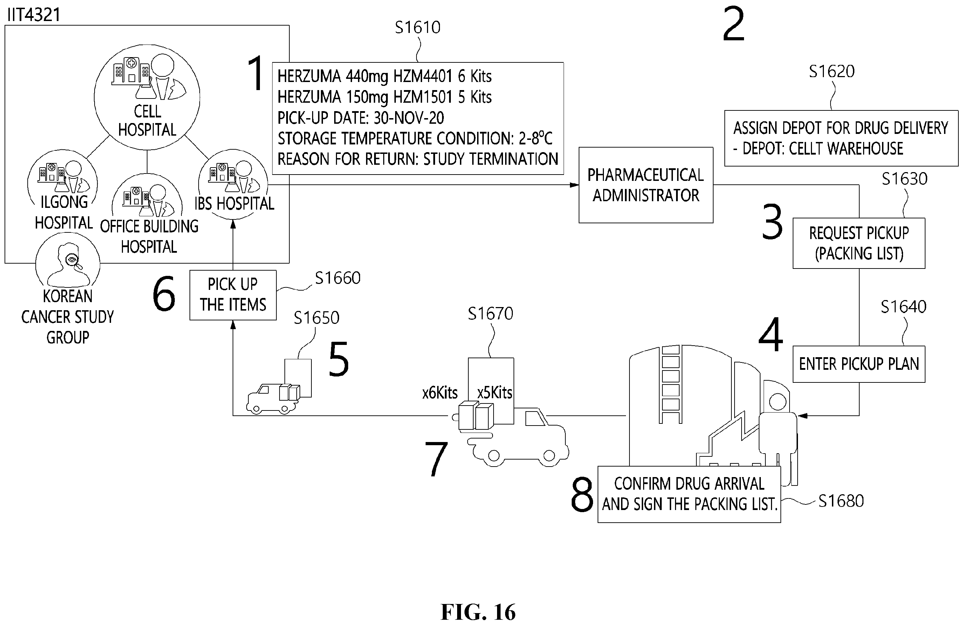

FIG. 16 illustrates an example of returning items using a clinical supplies management apparatus according to an embodiment of the present disclosure.

Referring to FIG. 16, it can be seen that a request for item return is made by a user of a study center type or a study monitor type. According to the embodiment, a user of the pharmaceutical administrator type may also be able to request a return of the items.

First, a request for item return may be generated by a user of the study center type, the study monitor type, or the pharmaceutical administrator type at step S1610.

Here, the supplies management apparatus according to an embodiment of the present disclosure may receive a request for return of items through an interface displaying information of ordering at least N (natural number) business days before the date of return required.

Here, in order to create a request for return items, information on the reason for return, information on whether the transportation temperature is controlled at the time of return, information on the place of origin, information on the items to be returned (items, dosage, return batch information and return quantity, etc.), pick-up request date information, etc. may be entered. Here, the reason for return information may be entered by selecting one item from a predetermined drop-down menu, such as clinical termination, expiration of expiration date, damage and temperature deviation, recall due to drug abnormalities, etc., or may be entered by a direct input method. Here, it may be determined whether to deduct the contract quantity according to the input return reason information. Here, the information on whether the transportation temperature is controlled at the time of return may be automatically determined according to the information of the reason for return or the information of the items to be returned. Here, the origin information may be automatically extracted from the account information of the user requesting the return of the items. Here, the item of the item information to be returned, the quantitative information, etc., may be entered through an interface in which the item information corresponding to the user requesting the return (which has a history of receipt at the corresponding study center) is automatically extracted and provided in the form of a drop-down. Here, the return batch information may be entered through an interface in which a batch list with a history of receiving from the study center is provided in the form of a drop-down. Here, the return quantity may be specified within the range of the amount supplied by the study center.

When a return request is generated, the supplies management apparatus according to an embodiment of the present disclosure may assign a depot to deliver the items at step S1620.

Here, the return number corresponding to the return request may be assigned according to the predetermined logic.

Here, an appropriate depot may be automatically assigned in consideration of the test authority or national authority of the registered depots of the system.

When the allocation of the return number and the depot corresponding to the return is completed, the clinical supplies management device according to an embodiment of the present disclosure may send an alarm email to a user of the pharmaceutical administrator type.

A user of the pharmaceutical administrator type who receives the alarm email can check the status of the item return request in the system and approve the return request if there is no abnormality.

A return request approved by a user of the pharmaceutical administrator type may be sent to a user of the depot type as a pickup request in the form of a packing list at step S1630.

Here, an alarm e-mail including the URL of the web page for entering the return plan may be sent to the depot type user who receives the pickup request.

A user of the depot type who receives a pickup request may enter a return plan (pickup plan) through a web page provided by a clinical supplies management device according to an embodiment of the present disclosure at step S1640.

Here, the input of the return plan may be performed not only by a user of the depot type but also by a user of the pharmaceutical administrator type.

Here, the return plan may include the pick-up date, the name of the courier company and the tracking number.

Here, if the return plan is not entered within a predetermined period after step S1630, the clinical supplies management device according to an embodiment of the present disclosure may send an alarm email to a user of the depot type and a user of the pharmaceutical administrator type.

When the input of the return plan is completed at step S1640, an alarm email may be sent to the user of the study center type, the study monitor type, the depot type, and / or the pharmaceutical administrator type. Here, a packing list may be attached to the alarm e-mail in the form of an attachment such as a PDF file so that it can be printed and used immediately at the receiving study center.

The return of the items (drugs) is carried out at step S1650, S 1660, S 1670 corresponding to the input of the return plan at step S 1640.

Here, the return of the items may be performed directly by a user of the depot type or by a courier.

When the returned item arrives at depot, the user of the depot type or the pharmaceutical administrator type enters the return completion information at step S 1680. Here, the return completion information may include POD information, item damage information, and temperature record information. The temperature record information may include temperature excursion and temperature data information. Here, the temperature data information may be registered as a file.

If there is a problem with the returned item (for example, when the temperature excursion information is set to Y, etc.), the item status update may be performed in consultation with the study center type, the study manager type or the depot type user and the pharmaceutical administrator type user for the problematic item.

When the return completion information is entered without any problems, the return process is terminated after updating the information on the returned volume.

If the return confirmation is not received after the predetermined date after the pick-up date included in the recall plan entered in step S1640, an alarm email may be sent to the user of the depot type and / or the pharmaceutical administrator type.

A user accessing a clinical supplies management device according to an embodiment of the present disclosure may access a clinical supplies management device through an OR code included in a distributed document.

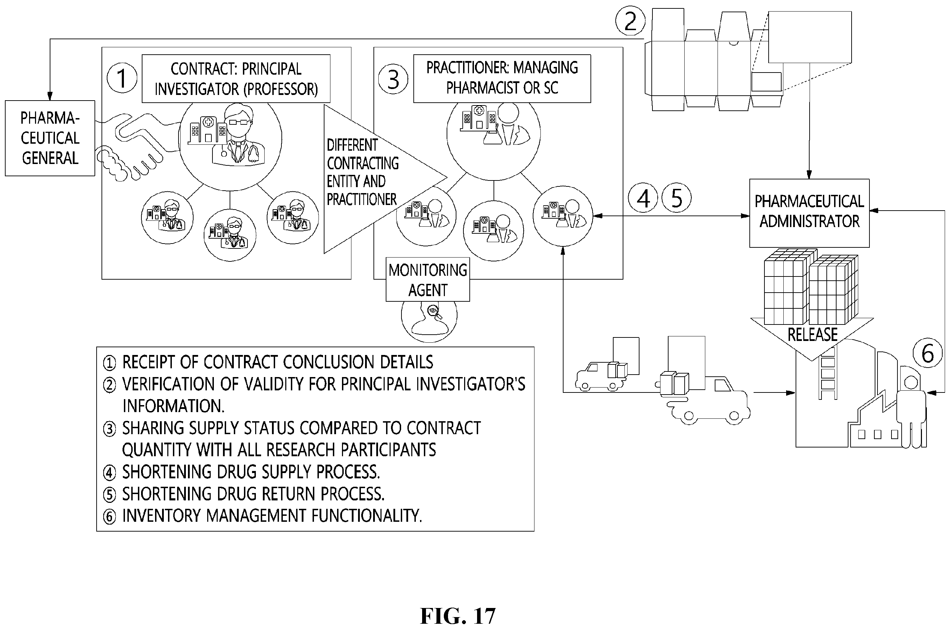

FIG. 17 illustrates an example of an overall process of managing supplies using a clinical supplies management apparatus according to an embodiment of the present disclosure.

Referring to FIG. 17, it can be seen that the clinical supplies management apparatus according to an embodiment of the present disclosure can effectively receive contract conclusion details from researchers.

In addition, in the clinical supplies management device according to an embodiment of the present disclosure, variable text information such as a person approved for clinical trials (principal investigator ) is confirmed at an appropriate time by the contracting entity, so that the accuracy of the information printed on the product packaging is guaranteed.

In addition, the clinical trial management device according to an embodiment of the present disclosure allows the user participating in the clinical trial to share the cumulative consumption status of the entire clinical quota not only for himself or herself but also for all users participating in the clinical trial, so that the efficiency of the investigator initiated clinical trial is maximized.

In addition, the clinical supplies management apparatus according to an embodiment of the present disclosure can maximize the efficiency of supplying and returning items required for clinical supplies management by appropriately managing the item supply or item return process through providing an appropriate email alarm or input interface.

In addition, the clinical supplies management apparatus according to an embodiment of the present disclosure can accurately and efficiently manage the quantity and state of items (drugs) in particular in depot. Furthermore, the clinical supplies management device according to an embodiment of the present disclosure may provide information on the contract conclusion status / history, transportation status, contract execution status and return status at a glance to a user corresponding to the pharmaceutical administrator type.

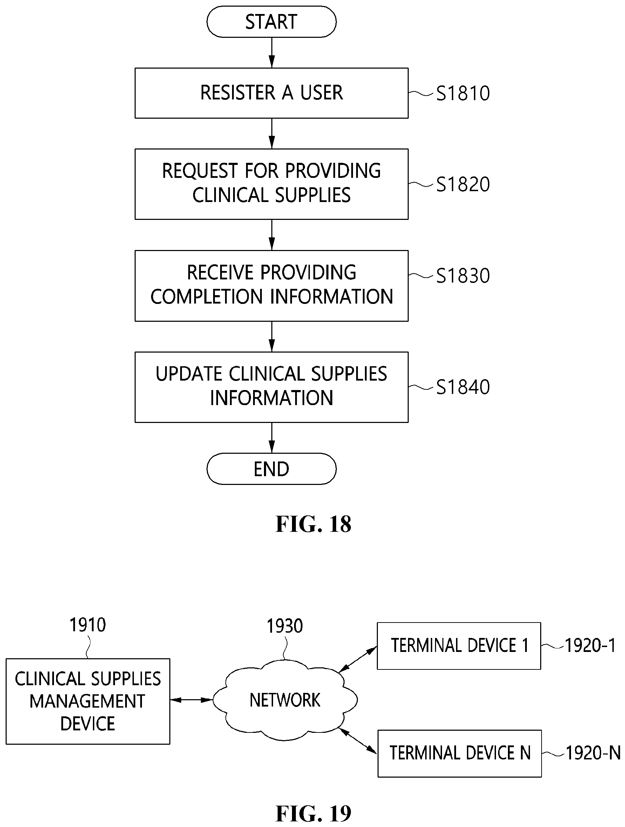

FIG. 18 is operation flow diagram illustrating an example of a clinical supplies management method according to an embodiment of the present disclosure.

Referring to FIG. 18, a method for clinical supplies management according to an embodiment of the present disclosure registers a user associated with a clinical trial according to user types at step S 1810.

Here, the user types may include study center type, study monitor type, depot type, pharmaceutical general type, and pharmaceutical administrator type.

In addition, the method for clinical supplies management according to an embodiment of the present disclosure receives a request for providing clinical supplies related to the clinical trial at step S 1820.

In addition, the method for clinical supplies management according to an embodiment of the present disclosure receives providing completion information for the clinical supplies at step S1830.

Here, the providing completion information may include temperature excursion information and physical damage information corresponding to the clinical supplies.

In addition, the method for clinical supplies management according to an embodiment of the present disclosure updates clinical supplies information corresponding to the providing completion information at step S1840.

Not shown in FIG. 18, the method for clinical supplies management according to an embodiment of the present disclosure may further include providing supply consumption status sharing interface for sharing the consumption status of the entire supplies allocated to the clinical trial to participants involved in the clinical trial.

Here, the supplies consumption status sharing interface may include graphs representing the total cumulative order quantities of the entire participants involved in the clinical trial, the total cumulative delivery completion quantities of the entire participants, and the total delivery quantities in progress of the entire participants.

Here, the supply consumption status sharing interface may be provided only for user types other than the depot type among the user types.

Not shown in FIG. 18, the method for clinical supplies management according to an embodiment of the present disclosure may further include receiving a secondary user registration request for registering another user associated with the clinical trial by users corresponding to the study center type or the study monitor type.

Not shown in FIG. 18, the method for clinical supplies management may further include receiving contract information corresponding to the contract related to the clinical trial. Here, the contract information may include investigator information related to the clinical trial, clinical supplies information, and information on individuals who have received approval for the clinical trial plan of the clinical trial.

Not shown in FIG. 18, the method for clinical supplies management may further include requesting confirmation of variable text information to be displayed on the packaging of the clinical supplies.

Here, the confirmation of variable text information may be requested from pharmaceutical administrator type user to pharmaceutical general type user. Here, the variable text information may include the information on individuals who have received approval for the clinical trial plan of the clinical trial.

Not shown in FIG. 18, the method for clinical supplies management may further include receiving a return request corresponding to the clinical supplies. Here, the return request may include information on the reason for return, information on whether the transportation temperature is controlled during return, departure point information, and supplies information to be returned. Here, the information on the reason for return may include any one or more of the clinical trial termination, expiration date expired, breakage/ temperature excursion, and recall due to drug abnormalities.

The clinical supplies management method according to an embodiment of the present disclosure may be implemented in the form of a program instruction that can be performed through various computer means and recorded on a computer-readable medium. The computer-readable medium may include program instructions, data files, data structures, and the like, alone or in combination. The program instructions recorded in the medium may be specifically designed and configured for the present disclosure or may be known and available to those skilled in the art in the computer software. Examples of computer-readable recording media include magnetic media such as hard disks, floppy disks, and magnetic tapes, optical media such as CD-ROMs and DVDs, magneto-optical media such as floptical disks, and any type of hardware device specifically configured to store and execute program commands such as ROMs, RAM, flash memory, and the like. Examples of program instructions may include machine code such as those generated by compilers, as well as high-level language code that can be executed by a computer using an interpreter, and the like. Such a hardware device may be configured to operate as one or more software modules to perform the operation of the present disclosure, and vice versa.

FIG. 19 is a block diagram illustrating a clinical supplies management system according to an embodiment of the present disclosure.

Referring to FIG. 19, the clinical supplies management system according to an embodiment of the present disclosure includes a clinical supplies management apparatus 1910, terminal devices 1920-1, ..., 1920-N, and a network 1930.

The clinical supplies management apparatus 1910 may perform functions for clinical supplies management described through FIGS. 1 to 17.

The clinical trial item management device 1920 may receive input necessary for performing a function from the terminal devices 1920-1, ..., 1920-N, and may provide the result of performing the function to the terminal devices 1920-1, ..., 1920-N.

The terminal devices 1920-1, ..., 1920-N receive an interface screen for clinical supplies management from the clinical supplies management apparatus 1910 and provide a study center type, a study monitor type, a depot type, a pharmaceutical general type, or a pharmaceutical administrator type.

Here, the terminal devices 1920-1, ..., 1920-N are connected to a communication network and can execute an application, and are not limited to a computer or a mobile communication terminal, but all information and communication devices, multimedia terminals, wired terminals, fixed terminals, and IP (Internet Protocol) terminals. In addition, the terminal devices 1920-1, ..., 1920-N are a mobile phone, a portable multimedia played (PMP), a mobile Internet device (MID), a smart phone, a desktop, a tablet computer, a notebook book, a net book, a personal digital assistant; PDA), smart TV, information and communication device, and the like may be a mobile terminal having various mobile communication specifications.

The network 1930 provides a channel for transmitting data between the clinical trial material management apparatus 1910 and the terminal devices 1920-1, ..., 1920-N, and is a concept that encompasses both the existing network and the network that can be developed in the future. For example, the network 1930 may consist of a wired or wireless short-range communication network that provides communication of various information devices within a limited area, a mobile communication network that provides communication between mobile bodies and between mobile objects and outside the mobile body, and a satellite communication network that provides communication between an earth station and a ground station using a satellite, or a combination of two or more of a wired and wireless communication network. On the other hand, the transmission method standard of the network 1930 is not limited to the existing transmission method standard and may include all transmission method standards to be developed in the future. In addition, in FIG. 19, the network used between the clinical supplies management apparatus 1910 and the terminal devices 1920-1, ..., 1920-N may be different from the network used between the terminal devices 1920-1, ..., 1920-N, or may be the same.

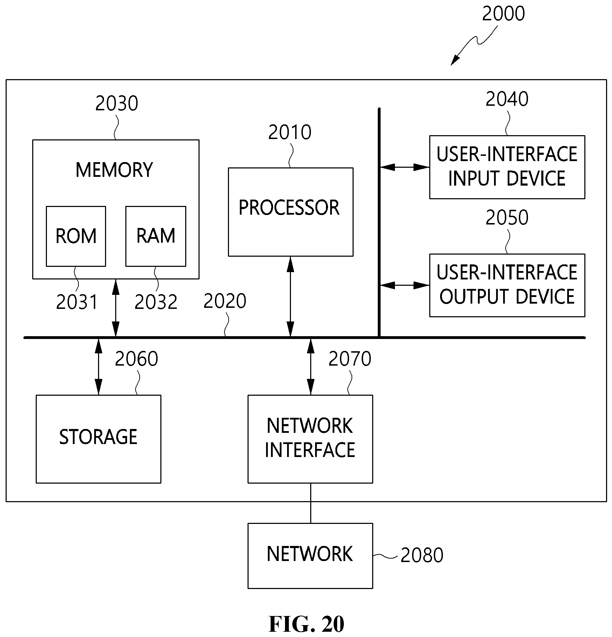

FIG. 20 illustrates a computer system according to an embodiment of the present disclosure.

Referring to FIG. 20, a clinical supplies management device (clinical supplies management device shown in FIG. 19) according to an embodiment of the present disclosure may be implemented in a computer system 2000 such as a computer-readable recording medium. As shown in FIG. 20, the computer system 2000 may include one or more processors 2010, memory 2030, user interface input device 2040, user interface output device 2050, and storage 2060 that communicate with each other via the bus 2020. In addition, the computer system 2000 may further include a network interface 2070 that connects to the network 2080. The processor 2010 may be a semiconductor device that executes processing instructions stored in the central processing unit or memory 2030 or storage 2060. The memory 2030 and the storage 2060 may be various forms of volatile or non-volatile storage media. For example, the memory may include ROM 2031 or RAM 2032.

The clinical supplies management apparatus according to an embodiment of the present disclosure includes one or more processors 2010, and memory 2030 for storing at least one program executed by one or more processors 2010, and the at least one of the programs may be for performing functions described through FIGS. 1 to 17.

Here, the processor 2010 may register a user associated with a clinical trial according to user types, receive a request for providing clinical supplies related to the clinical trial, receive providing completion information for the clinical supplies, and update clinical supplies information corresponding to the providing completion information.

Here, the memory 2030 may store the clinical supplies information.

Here, the processor 2010 may provide supply consumption status sharing interface for sharing the consumption status of the entire supplies allocated to the clinical trial to participants involved in the clinical trial.

Here, the supply consumption status sharing interface may include graphs representing the total cumulative order quantities of the entire participants involved in the clinical trial, the total cumulative delivery completion quantities of the entire participants, and the total delivery quantities in progress of the entire participants.

Here, the user types may include study center type, study monitor type, depot type, pharmaceutical general type, and pharmaceutical administrator type.

Here, the supply consumption status sharing interface may be provided only for user types other than the depot type among the user types.

Here, the processor 2010 may receive a secondary user registration request for registering another user associated with the clinical trial by users corresponding to the study center type or the study monitor type.

Here, the processor 2010 may request confirmation of variable text information to be displayed on the packaging of the clinical supplies. Here, the confirmation of variable text information may be requested from pharmaceutical administrator type user to pharmaceutical general type user. Here, the variable text information may include the information on individuals who have received approval for the clinical trial plan of the clinical trial.

As described above, the clinical supplies management apparatus and method according to the present disclosure may not be limited to the configuration and method of the embodiments described as described above, but the embodiments may be configured in an optional combination of all or part of each embodiment so that various modifications can be made.

## Claims

1. Clinical supplies management method performed by a clinical supplies management device, comprising:
registering a user associated with a clinical trial according to user types;
receiving a request for providing clinical supplies related to the clinical trial;
receiving providing completion information for the clinical supplies; and
updating clinical supplies information corresponding to the providing completion information.

2. The method of claim 1, further comprising:
providing supply consumption status sharing interface for sharing the consumption status of the entire supplies allocated to the clinical trial to participants involved in the clinical trial.

3. The method of claim 1, wherein the supply consumption status sharing interface includes graphs representing the total cumulative order quantities of the entire participants involved in the clinical trial, the total cumulative delivery completion quantities of the entire participants, and the total delivery quantities in progress of the entire participants.

4. The method of claim 2, wherein the user types include study center type, study monitor type, depot type, pharmaceutical general type, and pharmaceutical administrator type.

5. The method of claim 4, wherein the supply consumption status sharing interface is provided only for user types other than the depot type among the user types.

6. The method of claim 4, further comprising:
receiving a secondary user registration request for registering another user associated with the clinical trial by users corresponding to the study center type or the study monitor type.

7. The method of claim 2, wherein providing completion information includes temperature excursion information and physical damage information corresponding to the clinical supplies.

8. The method of claim 4, further comprising:
receiving contract information corresponding to the contract related to the clinical trial,
wherein the contract information includes investigator information related to the clinical trial, clinical supplies information, and information on individuals who have received approval for the clinical trial plan of the clinical trial.

9. The method of claim 8, further comprising:
requesting confirmation of variable text information to be displayed on the packaging of the clinical supplies.

10. The method of claim 8, wherein:
the confirmation of variable text information is requested from pharmaceutical administrator type user to pharmaceutical general type user, and
the variable text information includes the information on individuals who have received approval for the clinical trial plan of the clinical trial.

11. The method of claim 2, further comprising:
receiving a return request corresponding to the clinical supplies, wherein the return request includes information on the reason for return, information on whether the transportation temperature is controlled during return, departure point information, and supplies information to be returned.

12. The method of claim 11, wherein the information on the reason for return includes any one or more of the clinical trial termination, expiration date expired, breakage/ temperature excursion, and recall due to drug abnormalities.

13. A computer program stored on a computer-readable medium to execute the clinical supplies management method claimed in any one of claims 1 to 12.

14. A clinical supplies management device, comprising:
a processor that registers a user associated with a clinical trial according to user types, receives a request for providing clinical supplies related to the clinical trial, receives providing completion information for the clinical supplies, and updates clinical supplies information corresponding to the providing completion information; and
a memory that stores the clinical supplies information.

15. The clinical supplies management device of claim 14, wherein the processor provides supply consumption status sharing interface for sharing the consumption status of the entire supplies allocated to the clinical trial to participants involved in the clinical trial.

16. The clinical supplies management device of claim 15, wherein the supply consumption status sharing interface includes graphs representing the total cumulative order quantities of the entire participants involved in the clinical trial, the total cumulative delivery completion quantities of the entire participants, and the total delivery quantities in progress of the entire participants.

17. The clinical supplies management device of claim 15, wherein the user types include study center type, study monitor type, depot type, pharmaceutical general type, and pharmaceutical administrator type.

18. The clinical supplies management device of claim 17, wherein the supply consumption status sharing interface is provided only for user types other than the depot type among the user types.

19. The clinical supplies management device of claim 17, wherein the processor receives a secondary user registration request for registering another user associated with the clinical trial by users corresponding to the study center type or the study monitor type.

20. The clinical supplies management device of claim 17, wherein the processor requests confirmation of variable text information to be displayed on the packaging of the clinical supplies,
wherein:
the confirmation of variable text information is requested from pharmaceutical administrator type user to pharmaceutical general type user, and
the variable text information includes the information on individuals who have received approval for the clinical trial plan of the clinical trial.
